# EUROPEAN PATENT SPECIFICATION

(11) **EP 1 989 993 B1**
(45) Date of publication and mention of the grant of the patent: **30.05.2012**
(21) Application number: 08008461.9
(22) Date of filing: 05.05.2008
(51) Int. Cl.: A61B 1/01, A61B 1/012, A61B 1/12, A61M 25/00, A61M 25/01, A61M 25/10

(54) **Insertion assisting tool for an endoscope**
Tubus zur Einführung eines Endoskops
Tube à l'aide de l'insertion d'un endoscope

(30) Priority: 08.05.2007 JP 2007123832; 08.05.2007 JP 2007123834
(43) Date of publication of application: 12.11.2008
(73) Proprietor: FUJIFILM Corporation, Tokyo (JP)
(72) Inventor: Fujikura, Tetsuya, Saitama-shi Saitama (JP)
(74) Representative: Klunker . Schmitt-Nilson . Hirsch

(56) References cited:
- EP-A- 1 938 759
- EP-A1- 1 547 640
- WO-A-2007/081041
- DE-A1- 3 640 034
- US-A1- 2003 208 222

## Description

### BACKGROUND OF THE INVENTION

### 1. Field of the Invention

The present invention relates an insertion assisting tool and, in particular, relates to an insertion assisting tool for medical use which assists the insertion of an endoscope for observing a small intestine and a large intestine etc. into a human body. Further, the invention relates to an insertion assisting tool for an endoscope which includes an endoscope insertion portion to be inserted and a balloon provided at the outer periphery portion of the tip end thereof.

### 2. Description of Related Art

Since the deep portion of an alimentary canal such as a small intestine and a large intestine is bent complicatedly, the force is difficult to transmit to the tip end of the insertion portion by merely pushing the insertion potion of the endoscope into the deep portion of the alimentary canal, and so the insertion into the deep portion is difficult. Thus, there is proposed a method in which the insertion portion of the endoscope is inserted into an insertion assisting tool of a tubular shape (also called as an over tube or a sliding tube) and then inserted into a human body. According to this method, since the insertion portion is guided by the insertion assisting tool, the insertion portion can be prevented from excessively bending or curving and so can be inserted into the deep portion of the alimentary canal (see JP-A-10-248794, for example).

JP-A-2002-301019 discloses an endoscope apparatus in which a first balloon is provided at the tip end of the insertion portion of the endoscope and a second balloon is provided at the tip end of the insertion assisting tool. According to this endoscope apparatus, the insertion portion and the insertion assisting tool can be fixed at the alimentary canal by inflating the first balloon and the second balloon. Thus, the insertion portion can be inserted into the deep portion of the alimentary canal by alternatively inserting the insertion portion and the insertion assisting tool while repeatedly inflating and shrinking the balloons.

As such an insertion assisting tool, there is known one in which a tube for fluid is formed in the insertion assisting tool. In particular, a fluid tube provided with an opening portion at the outer periphery of the insertion assisting tool is sufficient so long as a path communicating to the opening portion is formed at the outer periphery from the base end side and it is not necessary to provide the tube to the tip end side therefrom. Thus, as disclosed in JP-A-2002-301019, for example, a tube for fluid (an air supply tube 24 for a second balloon) extending from the hand side to the opening portion is pasted oh the outer periphery of the insertion assisting tool. An air supply tube 56 disclosed in a patent document 2 has the similar configuration. Further, Japanese Patent No. 3804068 does not disclose the concrete structure of an air tube 82 for extracting air whether or not it is configured by pasting a tube provided separately from an insertion assisting tool on the outer surface of the insertion assisting tool later or it is integrally formed with the insertion assisting tool. Although an air tube 88 for extracting air of Japanese Patent No. 3804068 is integrally formed with the insertion assisting tool, since it is configured to penetrate to the tip end of the insertion assisting tool and doe not have an opening portion at the outer periphery thereof, it is not necessary to close the tip end side thereof.

The air supply tube of JP-A-2002-301019 is configured in a manner that an air passing tube is formed by pasting a tube having a small diameter on the outer surface of a tube main body serving as a guide of an endoscope insertion portion However, in this case, since the rigidity of the entirety of the insertion assisting tool is enhanced, the insertion assisting tool becomes difficult to bend, whereby it becomes difficult to insert the insertion assisting tool into a human body and also difficult to guide the endoscope insertion portion

In the case of combining the structures of the air tubes 82, 88 for extracting air in Japanese Patent No. 3804068, it is considered that a fluid tube is integrally formed with the insertion assisting tool to thereby form a path communicating from the base end side to the opening portion of the outer surface. However, in this case, the fluid tube is configured in a manner that the fluid tube communicates communicating from the base end side to the position of the opening portion of the outer surface and the fluid tube is bent to the direction of the outer periphery at the position of the opening portion. Thus, a complicated process is required since the tube is bent on the way thereof.

In the double balloon type endoscope apparatus disclosed in JP-A-2002-301019, when, for example, the second balloon is inflated and closely fitted to the intestinal wall and thereafter, an operation of moving the over tube in the extracting direction is performed, the over tube cannot be smoothly moved. Namely, this is considered to result from addition of compression to the air stored at a base end part side of the over tube with respect to the second balloon (air stored in a gap between the over tube and the intestinal wall) by the operation of the over tube, and the air pressure caused by this gives a difficulty to the extracting operation of the over tube.

In contrast, an insertion assisting tool disclosed in Japanese Patent No. 3804068 is arranged in a manner that a ventilation hole is provided on the base end side of a second balloon attaching position of the insertion assisting tool and the ventilation hole is opened to the base end portion of the insertion assisting tool via an air ventilation tube provided separately from an insertion path for the insertion assisting tool.

According to the insertion assisting tool, when the insertion assisting tool is extracted in the inflated state of the second balloon of the insertion assisting tool, the air stored in the gap between the insertion assisting tool and the intestinal wall enters from the ventilation hole of the insertion assisting tool and is exhausted to the outside of a human body via the insertion assisting tool. Thus, the insertion assisting tool is not applied with the air pressure at the time of the extracting operation of the insertion assisting tool. Accordingly, the extracting operation of the insertion assisting tool can be performed smoothly.

However, also in the insertion assisting tool for an endoscope having the ventilation hole disclosed in Japanese Patent No. 3804068, there arises a case that the air stored in the gap between the insertion assisting tool and the intestinal wall is not smoothly exhausted to the outside of a human body from the ventilation hole.

From WO 2007/081041 A1 an overtube having two inflatable balloons is known, between which a gas-supplying passage for supplying gas into a trachea of a patient is provided. From DE 36 40 034 A1 an esophagus probe is known having two inflatable balloons between which an opening for a treatment tool is provided. From US 2003/0208222 A1 a catheter for use in an emboli containment system is known. Further, from EP 1 938 759, a gastric therapy system with a loop-forming member is known, which forms a loop along a lesser-curvature-line and a greater-curvature line in a stomach.

### SUMMARY OF THE INVENTION

The invention is made in view of the aforesaid circumstances and an object of an illustrative embodiment of the invention is to provide an insertion assisting tool which is good in operability and can be easily processed..

Another object of an illustrative, non-limiting embodiment of the invention is to provide, in an insertion assisting tool having a ventilation hole, an insertion assisting tool for an endoscope which can surely exhaust air or liquid stored in a gap between the insertion assisting tool and an intestinal wall via the ventilation hole as an opening portion.

The aforesaid object can b e attained by the following aspects.
(1) An insertion assisting tool according to claim 1. In the insertion assisting tool according to (1), the opening has an elongated shape, and a longitudinal direction of the elongated shape is along an axial direction of the fluid tube. In the insertion assisting tool according to (1), an edge of the opening is subjected to a chamfer processing.
(2) In the insertion assisting tool according to (1), the fluid tube is closed by pressing the tube of the multi-lumen tube from the outside and by subjecting the tube to a heat processing.
(3) In the insertion assisting tool according to (1), the fluid tube is closed at a fixing position where a tip end of the balloon is fixed.
(4) In the insertion assisting tool according to (3), the outer peripheral surface is provide with a concave portion at a position where the fluid tube is closed, and the concave portion locates at the fixing position where the tip end of the balloon is fixed.
(5) In the insertion assisting tool according to (1), the position where the fluid tube is closed locates on the base end surface side than the position where the balloon is disposed.
(6) In the insertion assisting tool according to (1), the multi-lumen tube has a plurality of openings with an interval along the fluid tube.

According to the aspect (1), since the multi-lumen tube integrally formed by the insertion path and the tube is employed, as compared with the case where tubes separated prepared are pasted, the entire flexibility of the insertion assisting tool can be improved. Further, not only the operability at the time of inserting and drawing the insertion portion of the endoscope but also the operability at the time of inserting and drawing the insertion assisting tool within a human body can be improved.

Further, according to the aspect (1), since the opening is formed at the multi-lumen tube and the tube on the tip end side of the opening is closed, the insertion assisting tool can be manufactured easily.

Further, according to the aspect (2), since the tube is closed by pressing the tube of the multi-lumen tube from the outside and by subjecting the tube to the heat processing, the tube can be closed easily at an arbitrary position. For example, the tube can be closed near the opening.

Further, according to the aspect (2), since the tube is closed by pressing the tube from the outside and by subjecting the tube to the heat processing, the concave portion can be formed at the closing position. For example, the concave portion can be set as an index for positioning the balloon. Further, when the concave portion is set at the fixing position of the end portion of the balloon, the end portion of the balloon can be fixed firmly and so the balloon can be prevented from being detached and deviated, for example.

Further, according to the aspects (3) and (4), since the closing position of the tube is set at the fixing position of the end portion of the balloon, the closing position of the tube can act as an index of the fixing position of the end portion of the balloon. Thus, the end portion of the balloon can be fixed accurately at a desired position. Further, according to the aforesaid modes (3) and (4), the closing processing of the tube can be performed simultaneously with the processing for the index of the fixing position of the end portion of the balloon.

Furthermore, according to the aspect (5), since the tube for ventilation is closed at the position on the base end surface side than the disposing position of the balloon, the tube can be closed near the opening. Thus, the fluid etc. entered from the opening can be prevented from entering into the tube at the position where the balloon is disposed.

According to the aspect (1), the opening acting as a ventilation opening is formed to have the elongated shape, the longitudinal direction of which is along the longitudinal direction of the fluid tube.

In the insertion assisting tool disclosed in Japanese Patent No. 3804068, the reason why air stored between the insertion assisting tool and the intestinal wall can not be exhausted smoothly from the ventilation hole is that the ventilation hole is closed by the intestinal wall. Thus, the basic reason why the ventilation hole is closed by the intestinal wall has been investigated and found that the ventilation hole of Japanese Patent No. 3804068 is a perfect circle and the basic reason causing such a problem resides in the shape of the ventilation hole.

According to the aspect (1), in the case where the inner wall of the body cavity adheres to the opening portion, since the opening portion is formed in the elongated shape, the entirety of the opening portion can be more unlikely closed as compared with the opening portion of the perfect circle. Thus, since the exhausting function of the opening portion can be maintained, the air and liquid reserved between the insertion assisting tool and the intestinal wall can be surely exhausted to the outside of a human body from the opening portion.

According to the aspect (6), of the plurality of the opening portions, even if one of the opening portions is closed by the inner wall of the body cavity adhering thereto, air and liquid can be exhausted to the outside of a human body from the remaining opening portion not being closed.

According to the aspect (1), since the opening area of the opening portion is set to be larger than the sectional area of the tube orthogonal to the axial direction of a fluid path, the exhausting efficiency of air and liquid can be improved.

According to the aspect (1), since the edge of the opening portion is subjected to the chamfering processing, the inner wall of the body cavity can be prevented from being caught by the opening portion, so that the operability of inserting and extracting the insertion assisting tool with respect to the inner wall of the body cavity can be improved. Thus, pain of a patient due to the catch of the inner wall of the body cavity by the opening portion can be relieved. Further, the edge of the opening portion is an edge on the outer surface side of the insertion assisting tool and at least the edge almost orthogonal to the insertion and extracting direction of the insertion assisting tool may be subjected to the chamfering processing.

### BRIEF DESCRIPTION OF THE DRAWINGS

The features of the invention will appear more fully upon consideration of the exemplary embodiment of the invention, which are schematically set forth in the drawings, in which:
Fig. 1 shows a system configuration of an endoscope according to an exemplary embodiment of the invention;
Fig. 2 is a perspective view showing the tip end portion of the insertion portion of the endoscope;
Fig. 3 is a front view showing an insertion assisting tool;
Fig. 4 is a plan view showing the tip end side of a tube main body;
Fig. 5 is a bottom view showing the tip end side of the tube main body;
Fig. 6 is a longitudinal sectional view showing the tip end side of the tube main body;
Fig. 7 is a sectional view of the tube main body along a line VII-VII in Fig. 3;
Fig. 8 is a diagram for explaining the manufacturing method of the insertion assisting tool;
Fig. 9 is an explanatory diagram showing a drawn-out state of the insertion assisting tool;
Fig. 10 is an explanatory diagram for comparing the sectional area of an opening portion with a sectional area of a tube;
Fig. 11 is an enlarged sectional diagram showing a main portion of the opening portion; and
Figs. 12A and 12B are explanatory diagrams showing an example of a processing method of the opening portion,
wherein some of the reference numerals and signs in the drawings are set forth below.
- 10: endoscope
- 12: insertion portion
- 14: hand side operating portion
- 60: insertion assisting tool
- 62: griping portion
- 64: tube main body
- 66: balloon
- 70: insertion path
- 72: tube
- 74: tube
- 80: opening
- 82: concave portion
- 84: concave portion
- 90: opening

### DETAILED DESCRIPTION OF THE EXEMPLARY EMBODIMENTS

According to an exemplary embodiment of the invention, since the multi-lumen tube integrally formed by the insertion path and the tube is employed and the tube of the multi-lumen tube is closed on the tip end portion side than the opening, the insertion assisting tool can be processed easily and the entire flexibility of the insertion assisting tool can be improved. Further, not only the operability at the time of inserting and drawing the insertion portion of the endoscope but also the operability at the time of inserting and drawing the insertion assisting tool within a human body can be improved.

Further, according to another embodiment, since the opening portion acting as a ventilation hole is formed in the elongated shape which longitudinal direction is along the axial direction of the insertion assisting tool, the entirety of the opening portion can be more unlikely closed as compared with the opening portion of the perfect circle. Thus, since the exhausting function of the opening portion can be maintained, the air and liquid reserved between the insertion assisting tool and the intestinal wall can be surely exhausted to the outside of a human body from the opening portion.

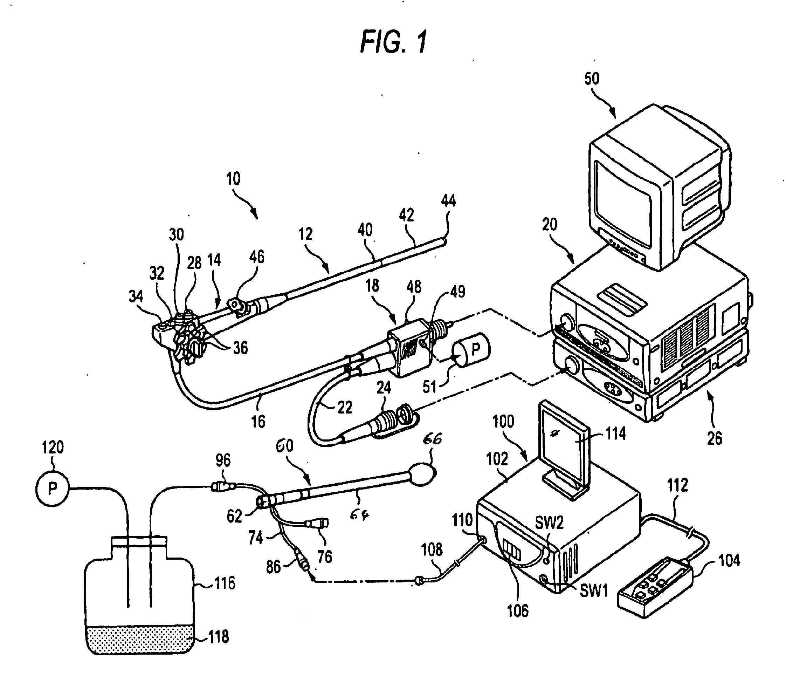

Hereinafter, the insertion assisting tool according to an exemplary embodiment of the invention will be explained with reference to attached drawings. Fig. 1 is a system configuration diagram showing an endoscope apparatus to which the insertion assisting tool according to the invention is applied. As shown in Fig. 1, the endoscope apparatus is mainly configured by an endoscope 10, an insertion assisting tool 60 and a balloon control device 100.

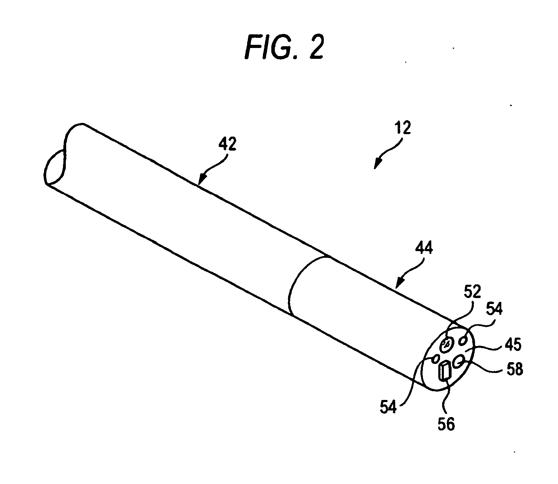

The endoscope 10 includes a hand side operating portion 14 and an insertion portion 12 which is provided so as to continue from the hand side operating portion 14 and inserted within a human body. A universal cable 16 is coupled to the hand side operating portion 14 and an LG connector 18 is provided at the tip end of the universal cable 16. The LG connector 18 is coupled to a light source device 20 so as to be detachable freely, whereby illumination light is transmitted to illumination optical systems 54 described later (see Fig. 2). An electric connector 24 is coupled to the LG connector 18 via a cable 22 and the electric connector 24 is coupled to a processor 26 so as to be detachable freely.

The hand side operating portion 14 is provided with an air supply/liquid supply button 28, a suction button 30, a shutter button 32 and a function switching button 34and further provided with a pair of angle knobs 36, 36.

The insertion portion 12 is configured by a soft portion 40, a curved potion 42 and a tip end portion 44 in the order from the hand side operating portion 14 side. The curved potion 42 is remotely operated so as to curve by rotating the angle knobs 36, 36 of the hand side operating portion 14, whereby the tip end portion 44 can be directed to a desired direction.

As shown in Fig. 2, the tip end surface 45 of the tip end portion 44 is provided with an observation optical system 52, the illumination optical systems 54, 54, an air supply/liquid supply nozzle 56 and a forceps port 58. A CCD (not shown) is disposed on the rear side of the observation optical system 52. A signal cable (not shown) is coupled to a board for supporting the CCD. The signal cable is inserted into the insertion portion 12, the hand side operating portion 14 and the universal cable 16 etc. shown in Fig. 1, then extended to the electric connector 24 and coupled to the processor 26. Thus, an observed image taken at the observation optical system 52 is focused on the light receiving surface of the CCD and converted into an electric signal. The electric signal is inputted into the processor 26 via the signal cable and converted into an image signal, whereby the observed image is displayed on a monitor 50 coupled to the processor 26.

The outgoing light end of a light guide (not shown) is disposed on the rear side of the illumination optical systems 54, 54. The light guide is inserted into the insertion portion 12, the hand side operating portion 14 and the universal cable 16 shown in Fig. 1 and the incident light end thereof is disposed within the LG connector 18. Thus, when the LG connector 18 is coupled to the light source device 20, illumination light emitted from the light source device 20 is transmitted to the illumination optical systems 54, 54 via the light guide and irradiated in the forward direction from the illumination optical systems 54, 54.

The air supply/liquid supply nozzle 56 shown in Fig. 2 communicates with a valve (not shown) which is operated by the air supply/liquid supply button 28 shown in Fig. 1. The valve communicates with an air supply/liquid supply connector 48 provided at the LG connector 18. The air supply/liquid supply connector 48 is coupled to a not-shown air supply/water supply means and so supplied with air and water therefrom. The air supply/liquid supply nozzle 56 is disposed so as to be directed to the observation optical system 52. Thus, the air and water can be injected toward the observation optical system 52 from the air supply/liquid supply nozzle 56 by operating the air supply/liquid supply button 28.

The forceps port 58 shown in Fig. 2 communicates with a forceps insertion portion 46 shown in Fig. 1. A treatment tool such as a forceps can be extracted from the forceps port 58 by inserting the treatment tool from the forceps insertion portion 46. The forceps port 58 communicates with a valve (not-shown) operated by the suction button 30. This valve is coupled to the suction connector 49 of the LG connector 18. Thus, a diseased portion etc. can be sucked from the forceps port 58 when the valve is operated by using the suction button 30 after coupling a suction pump 51 to the suction connector 49.

On the other hand, the insertion assisting tool 60 shown in Fig. 1 is mainly configured by a gripping portion 62 and a tube main body 64 acting as a multi main tube. The gripping portion 62 is a portion gripped by an operator which is formed in a tubular shape by hard material such as plastics. The tube main body 64 is fitted on the outer periphery of the tip end side of the gripping portion 62 and fixed thereto.

The tube main body 64 is formed by elastic material such as polyurethane and is formed in an almost tubular shape in which the insertion portion 12 of the endoscope 10 is inserted. A balloon 66, which is freely inflatable and shrinkable, is attached to the outer periphery of the tip end portion of the tube main body 64. Air is supplied from the balloon control device 100 to the balloon 66 and sucked from the balloon by the balloon control device. The detail of the insertion assisting tool 60 will be described in detail later.

The balloon control device 100 shown in Fig. 1 is a device for supplying and sucking fluid such as air to and from the balloon 66, respectively, and is mainly configured by a device main body 102 and a hand switch 104 for remote control.

The device main body 102 is provide on the front surface thereof with a power supply switch SW1, a stop switch SW2 and a pressure display portion 106. The pressure display portion 106 is a panel for displaying a pressure value of the balloon 66, and an error code is displayed on the pressure display portion 106 at the time of occurrence of an abnormality such as a breakage of the balloon.

A tube (or a cable) 108 for supplying and sucking air to and from the balloon 66 is coupled to the front surface of the device main body 102. A reverse flow preventing unit110 is provided at the coupling portion between the tube 108 and the device main body 102. The reverse flow preventing unit110 is configured by incorporating an air/liquid separation filter within a casing (not shown) of a hollow disc shape detachably attached to the device main body 102. When the balloon 66 is broken, the filer can prevent liquid such as body fluid from flowing into the device main body 102.

The hand switch 104 is provided with various kinds of switches. For example, there are provided with a stop switch having the same function as the stop switch SW2 on the device main body 102 side, an ON/OFF switch for instructing the increase/decrease of the pressure within the balloon 66 and a pause switch for maintaining the pressure within the balloon 66. The hand switch 104 is electrically coupled to the device main body 102 via a cord 112. Although not shown in Fig. 1, the hand switch 104 is provided with a display portion for indicating the air supply state or the air exhaust state of the balloon 66.

The balloon control device 100 thus configured can supply air to the balloon 66 to inflate it, control the air pressure within the balloon at a constant value to hold the balloon 66 to the inflated state, suck air from the balloon 66 to shrink it, and control the air pressure within the balloon at a constant value to hold the balloon 66 to the shrunk state.

The balloon control device 100 is coupled to a balloon dedicated monitor 114, whereby at the time of inflating or shrinking the balloon 66, the pressure value and the inflated/shrunk state of the balloon 66 can be displayed on the balloon dedicated monitor 114. The pressure value and the inflated/shrunk state of the balloon 66 may be displayed on the monitor 50 in a manner of being superimposed on the observed image of the endoscope 10.

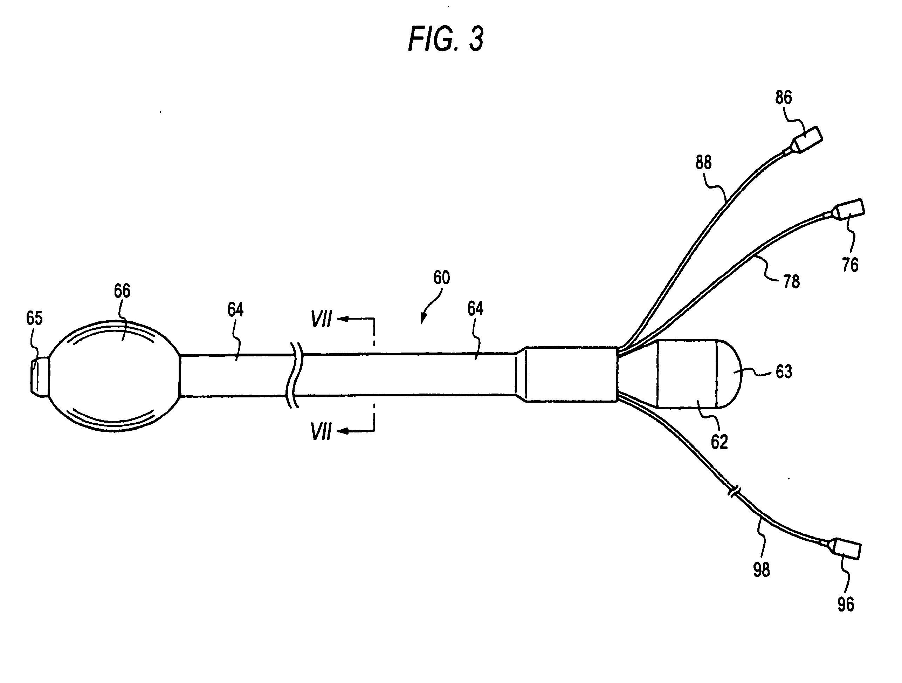

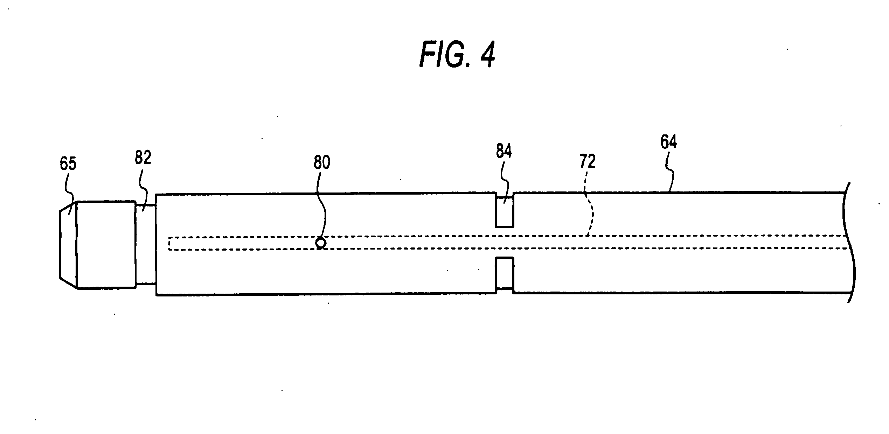

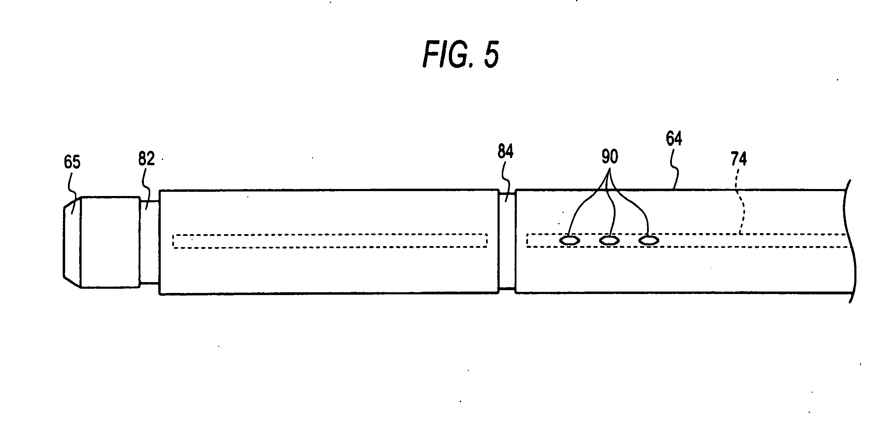

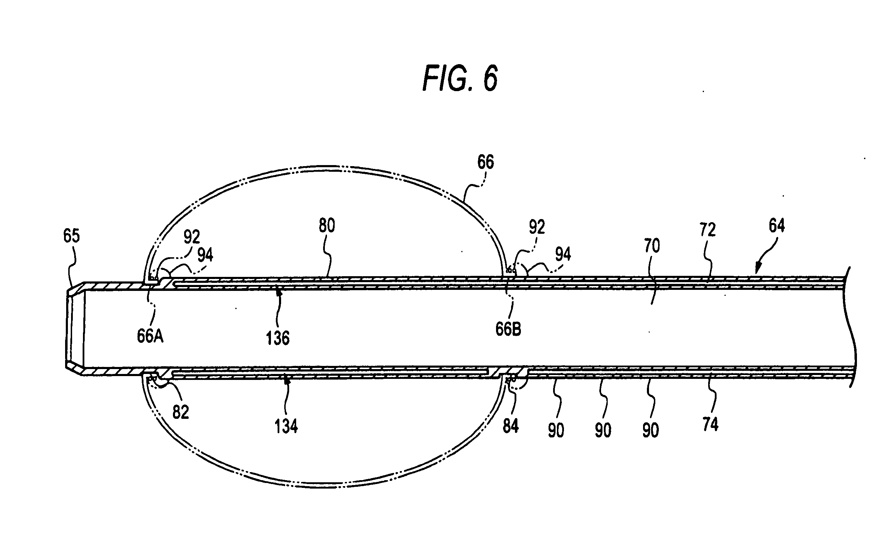

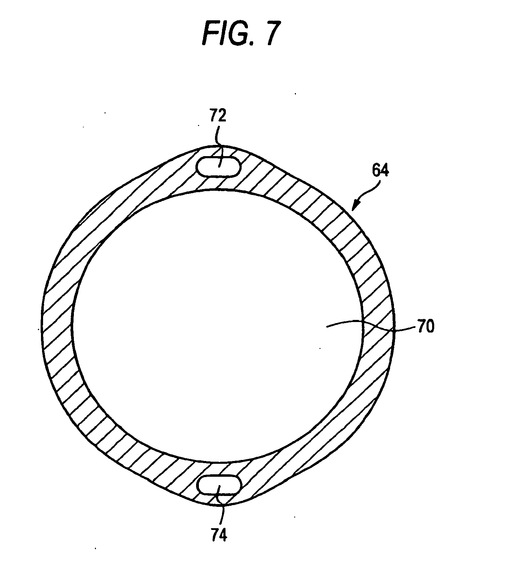

Next, the 60 according to the embodiment will be explained with reference to Figs. 3 to 7. Fig. 3 is a front view showing the insertion assisting tool 60 except for the balloon 66. Fig. 4 is a plan view showing the tip end portion of the tube main body 64. Fig. 5 is a bottom view showing the tip end portion of the tube main body 64. Fig. 6 is a longitudinal sectional diagram showing the tip end portion of the tube main body 64 including the balloon 66. Fig. 7 is a sectional view showing the tube main body 64 along a line VII-VII in Fig. 3.

As shown in Figs. 6 and 7, an insertion path 70, a fluid tube 72 for the balloon and a ventilation tube (fluid tube) 74 are formed so as to extend in the axial direction of the tube main body 64 within the tube main body 64.

The insertion path 70 is a tube into which the insertion portion 12 (see Fig. 1) of the endoscope 10 is inserted and is configured in a manner that the sectional shape thereof orthogonal to the axial direction is circle and the inner diameter is slightly larger than the outer diameter of the insertion portion 12. The inner peripheral surface of the insertion path 70 is coated with hydrophilic coating material (lubricative coating material) such as polyvinyl pyrrolidone. Thus, the friction between the tube main body 64 and the insertion portion 12 can be reduced by supplying lubricant such as water to the inner periphery of the insertion path 70 (that is, a space between the tube main body 64 and the insertion portion 12).
The lubricant can be supplied by injecting the lubricant by using a hypodermic syringe etc. (not shown) from a connector 76 shown in Fig. 3. The connector 76 is coupled to a tube 78 with a thin diameter, and the tip end of the tube 78 is coupled to the base end of the insertion path 70.

The fluid tube 72 for the balloon shown in Figs. 6 and 7 is a tube for supplying and sucking fluid (for example, air) to and from the balloon 66, respectively, and is integrally formed in the axial direction within the tube wall of the insertion path 70. As shown in Fig. 7, the fluid tube 72 for the balloon is configured in a manner that the sectional shape thereof orthogonal to the axial direction of the tube main body 64 is an ellipse which is short in the radial direction of the tube main body 64 and long in the circumferential direction. Accordingly, the tube main body 64 is suppressed from protruding outside while securing a sufficient flow path area of the tube 72. The sectional shape of the tube 72 is sufficient so long as it is short in the radial direction and long in the circumferential direction, and so may be bent in parallel to the inner peripheral surface of the insertion path 70.

The fluid tube 72 for the balloon shown in Figs. 6 and 7 is a tube for supplying and sucking fluid (for example, air) to and from the balloon 66, respectively, and is integrally formed in the axial direction within the tube wall of the insertion path 70. As shown in Fig. 7, the fluid tube 72 for the balloon is configured in a manner that the sectional shape thereof orthogonal to the axial direction of the tube main body 64 is an ellipse which is short in the radial direction of the tube main body 64 and long in the circumferential direction. Accordingly, the tube main body 64 is suppressed from protruding outside while securing a sufficient flow path area of the tube 72.

As shown in Figs. 4 and 6, the fluid tube 72 for the balloon communicates with an opening 80 for the balloon formed at the outer peripheral surface of the tube main body 64. The opening 80 for the balloon is formed at the attachment position of the balloon 66 (that is, the center position between concave portions 82 and 84 described later). The balloon 66 can be inflated and shrunk by supplying and sucking air to and from the opening 80, respectively.

The tip end side of the tube 72 is closed at the fixing portion of the tip end 66A of the balloon 66 and the tip end side than the fixing position. The base end side of the tube 72 is coupled to a tube 88 with a small diameter (see Fig. 3) at the base end position of the tube main body 64. A connector 86 is provided at the tip end of the tube 88. The fluid tube 72 for the balloon is coupled to the balloon control device 100 by coupling the connector 86 to the tube 108 shown in Fig. 1. Thus, the balloon 66 can be inflated and shrunk by supplying and sucking air by using the balloon control device 100.

The ventilation tube 74 shown in Figs. 6 and 7 is provided on the opposite side of the fluid tube 72 for the balloon via the insertion path 70 and is formed in the axial direction within the tube wall of the insertion path 70. Like the fluid tube 72 for the balloon, the ventilation tube 74 is configured in a manner that the sectional shape thereof shown in Fig.7 is an ellipse which is short in the radial direction and long in the circumferential direction. Accordingly, an outwardly protruding amount of the tube main body 64 can be suppressed while securing a sufficient flow path area of the tube 74. The sectional shape of the tube 74 is sufficient so long as it is short in the radial direction and long in the circumferential direction, and so may be bent in parallel to the inner peripheral surface of the insertion path 70.

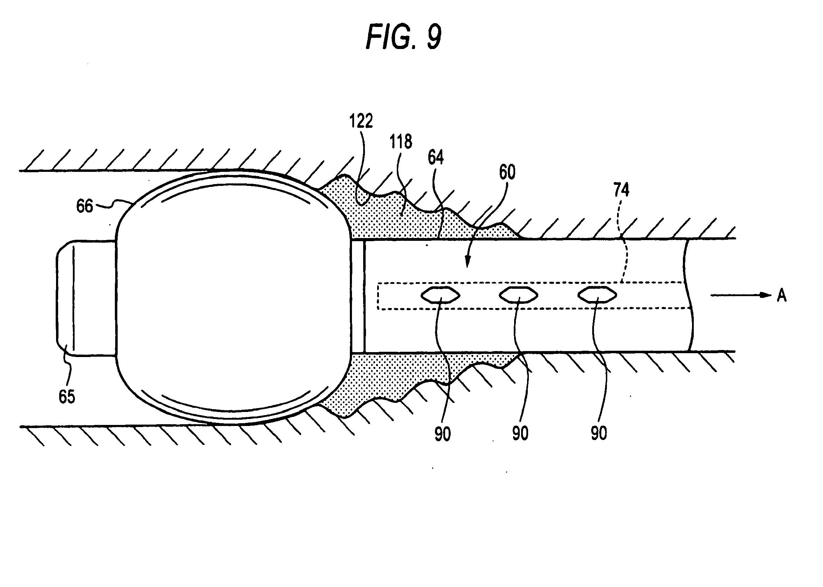

The ventilation tube 74 communicates with the outside via openings 90, 90, 90 for ventilation formed at the outer peripheral surface of the tube main body 64. The ventilation openings 90, 90, 90 are formed with a constant interval on the base end side than the attachment position of the balloon 66. As shown in Fig. 9, the opening area of each of the openings 90 is set to be equal to or larger than the sectional area of the tube 74 so that the sufficient ventilation can be realized even by the single opening 90.

As shown in Fig. 6, the tip end side of the tube 74 is closed at the fixing position of the base end portion 66B of the balloon 66. On the other hand, the base end side of the tube 74 is coupled to a tube 98 with a small diameter (see Fig. 3) at the base end position of the tube main body 64, whereby the tube 74 communicates with the outside via a connector 96 provided at the tip end of the tube 98, whereby the ventilation openings 90 are communicated with the outside via the tube 74 and the connector 96 and so the ventilation with the outside can be secured.

As shown in Fig. 1, the tube 98 is coupled to a tank 116, liquid 118 flown via the path 74 from the openings 90 can be reserved within the tank 116 from the tube 98. Further, when a suction pump 120 is coupled to the tank 116, the fluid 118 and air can be sucked forcedly.

As shown in Figs. 4 to 6, the two concave portions 82, 84 are formed with a predetermined interval therebetween at the attachment position of the balloon 66 on the outer peripheral surface of the tube main body 64. The concave portion 82 on the tip end side is formed along the entire circumferential periphery of the tube main body 64. On the other hand, as shown in Fig. 4, the concave portion 84 on the base end side is formed along the entire circumferential periphery of the tube main body except for an area near the fluid tube 72 for the balloon and is formed in a C shape. The tip end portion 66A and the base end portion 66B of the balloon 66 shown in Fig. 6 are fixed to the concave portions 82, 84, respectively.

The balloon 66 is formed in an almost tubular shape which center portion is expanded, and the tip end portion 66A of the balloon is covered by the concave portion 82 of the tube main body 64 in a reversed state thereof. A string 92 is wound around the tip end portion 66A of the balloon 66 and adhesive 94 is pasted on the string, so that the tip end portion 66A of the balloon 66 is fixed to the tube main body 64. In this state, the balloon 66 is restored into an original state and the base end portion 66B of the balloon 66 is covered by the concave portion 84. Then, a string 92 is wound around the base end portion 66B of the balloon 66 and adhesive 94 is pasted on the string, so that the base end portion 66B of the balloon 66 is fixed to the tube main body 64. In this manner, the tip end portion 66A and the base end portion 66B of the balloon 66 are fixed to the concave portions 82, 84, respectively. In this case, since the tip end portion 66A and the base end portion 66B of the balloon 66 are disposed at the concave portions 82, 84, respectively, the fixing portions are suppressed from protruding outwardly.

As shown in Figs. 4 to 6, a tapered portion 65 is formed at the tip end of the tube main body 64 in a manner that the diameter of the tube main body becomes smaller toward the tip end. Thus, when the insertion portion 12 of the endoscope 10 is inserted into the insertion path 70, since a gap between the insertion portion 12 and the tip end of the tube main body 64 becomes small, the lubricant can be suppressed from leaking to the tip end side of the tube main body 64.

As shown in Fig. 3, a tube 63 for preventing leakage is provided at the base end of the insertion assisting tool 60 (that is, the base end of the gripping portion 62). The leakage preventing tube 63 is formed by elastic material such as rubber and is configured in a shape which diameter becomes smaller as the leakage preventing tube becomes closer to the base end side (the right side in Fig. 3) of the insertion assisting tool 60, whereby the lubricant can be prevented from leaking since the gap between the insertion portion 12 and the leakage preventing tube becomes smaller.

The connectors 76, 86, 96 shown in Fig. 3 are preferably made different in their colors, sizes and shapes etc. from one another so as to be easily discriminated. In this case, the probability of the erroneous attachment of the respective tubes to the devices can be reduced. When the shapes of the connectors 76, 86, 96 are made different from one another, since the connector can not be attached to the erroneous device even if tried, the erroneous attachment can be prevented surely. Further, the tubes 78, 88, 98 may be made different in their lengths and colors etc. in place of differentiating the connectors 76, 86, 96.

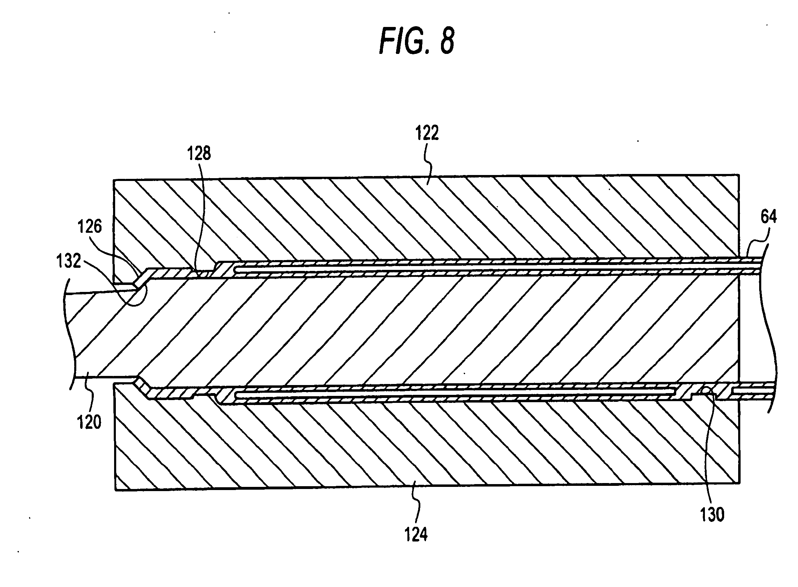

The tube main body 64 can be manufactured by processing a multi-lumen tube by using a mould shown in Fig. 8. The mould shown in Fig. 8 is configured by a cored bar 120 and two embossing dies 122, 124. The tip end portion of the cored bar 120 is formed to have an outer diameter almost same as the inner diameter of the insertion path 70 shown in Fig. 7. A tapered portion 126 is formed on the outer peripheral surface of the cored bar 120. The embossing dies 122, 124 are configured to form a tubular shape when faced to each other. These embossing dies are provided with two convex portions 128, 130 on the inner peripheral surfaces thereof. The convex portion 128 is protruded along the entire circumferential periphery of the embossing dies, whilst the convex portion 130 is protruded along the entire circumferential periphery of the embossing dies except for a potion and so configured in a C shape. Each of the embossing dies 122, 124 is provided with a tapered portion 132 having the same tapered angle with the tapered portion 126.

Although not shown, the multi-lumen tube before the processing has a tip end surface, a base end surface and an outer peripheral surface. After the processing, three holes to be the insertion path 70, the tube 72 and the tube 74 respectively are formed in the axial direction within the multi-lumen tube so as to penetrate between the tip end surface and the base end surface, and the sectional shape orthogonal to the axis is formed to be uniform always. First, the cored bar 120 is inserted into the multi-lumen tube and the embossing dies 122, 124 are pressed toward the multi-lumen tube from the outside. The embossing dies 122, 124 are heated to a predetermined temperature (for example, 100 to 110 degrees centigrade) while pressing the multi-lumen tube by the embossing dies 122, 124 to thereby form the tube main body 64 having the two concave portions 82, 84 and the tapered portion 65.

The tube main body 64 manufactured in this manner forms a space 134 closed by the two concave portions 82, 84 (that is, two closed portions) as shown in Fig. 6. The space 134 contains air confined therein and so more excellent flexibility can be obtained as compared with the case where the tube main body is processed so as to fill or eliminate the space. Thus, the insertion operability of the insertion assisting tool 60 into a human body and also the insertion operability of the insertion portion 12 into the insertion assisting tool 60 can be improved.

The tube main body 64 manufactured by the aforesaid manufacturing method is configured in a manner that the closing position of the fluid tube 72 for the balloon (that is, the concave portion 82) is separated from the opening 80 in the axial direction and the semi-closed space 136 is formed on the tip end side than the opening 80. Thus, when air is supplied via the tube 72, the semi-closed space 136 can absorb the pressure deviation of the air blown out from the opening 80.

Further, since the tube main body 64 is provided with the two concave portions 82, 84, these concave portions 82, 84 act as indices at the time of fixing the tip end portion 66A and the base end portion 66B of the balloon 66, so that the tip end portion 66A and the base end portion 66B of the balloon 66 can be surely fixed at the desired positions, respectively. Thus, it is possible to prevent such a phenomenon that the distance between the tip end portion 66A and the base end portion 66B of the balloon 66 is too short or too large, and hence the expansion coefficient of the balloon 66 can be controlled accurately. Further, since the tip end portion 66A and the base end portion 66B of the balloon 66 are fixed to the concave portions 82, 84, respectively, the concave portions 82, 84 act as catching portions for the fixing means such as the string 92 and the adhesive 94, the balloon 66 can be fixed firmly.

Next, the explanation will be made as to the operation method of the endoscope apparatus configured in the aforesaid manner. In the endoscope apparatus configured in the aforesaid manner, first the insertion portion 12 and the insertion assisting tool 60 are alternatively inserted in a pushing manner and the insertion assisting tool 60 is fixed within a cavity of a human body (for example, a large intestine) while inflating the balloon 66 if necessary. Then, the insertion assisting tool 60 is mobbed in the extracting direction to thereby simplify the tubular shape of the cavity (for example, a large intestine), and the insertion portion 12 is further inserted into the deep portion of the cavity. For example, the insertion portion 12 is inserted from the anus of a person to be tested, then the balloon 66 is inflated almost when the tip end of the insertion portion 12 passes a sigmoid colon, and the insertion assisting tool 60 is fixed to the intestinal canal. Then, the insertion assisting tool 60 is pulled to form the sigmoid colon in an almost straight shape, and the tip end of the insertion portion 12 is inserted into the deep portion. In this manner, the insertion portion 12 can be inserted into the deep portion of the sigmoid colon.

Of the aforesaid operations, in the operation for moving the insertion assisting tool 60 in the extracting direction in the inflated state of the balloon 66, as shown in Fig. 9, the air or the liquid 118 reserved between the tube main body 64 and the intestinal wall is exhausted to the outside via the tube 74 from the ventilation openings 90, 90, 90 of the tube main body 64. Thus, when the insertion assisting tool 60 is operated in the extracting direction, the air or liquid 118 reserved between the tube main body 64 and the intestinal wall 122 can be prevented from being compressed, so that the insertion assisting tool 60 can be smoothly operated in the extracting direction (an arrow A direction in Fig. 9).

The insertion assisting tool 60 according to the embodiment employs the multi-lumen tube having integrally-formed tubes as shown in Fig. 7, as the tube main body 64. Since the multi-lumen tube, in which the tubes are formed in advance, is used as the tube main body 64, these tubes can be used as the tubes 72, 74 as they are and so the tube main body 64 can be constituted easily.

As shown in Fig. 9, the three openings 90, 90, 90 are formed with the predetermined interval along the tube 74. Thus, even if the inner wall of the body cavity adheres to and close the one of these openings 90, 90, 90, the air and fluid can be surely exhausted to the outside from the remaining openings 90 not being closed. The number of the openings 90 is not limited to three and the number is sufficient so long as two or more.

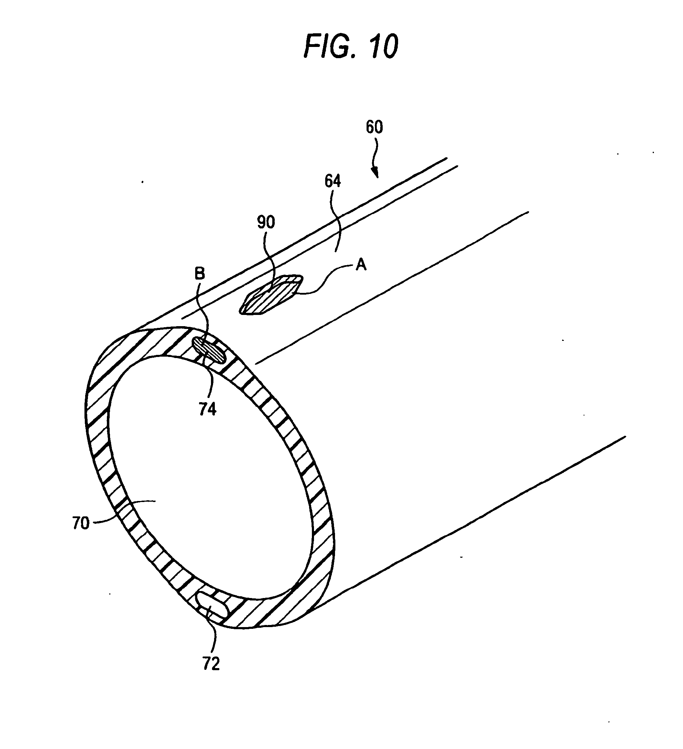

As shown in Fig. 10, an opening area A of each of the openings 90 shown by hatched lines is formed to be larger than a sectional area B of the tube 74 shown by hatched lines which is orthogonal to the longitudinal direction of the tube. Thus, the exhausting efficiency of the air and the fluid 118 can be improved.

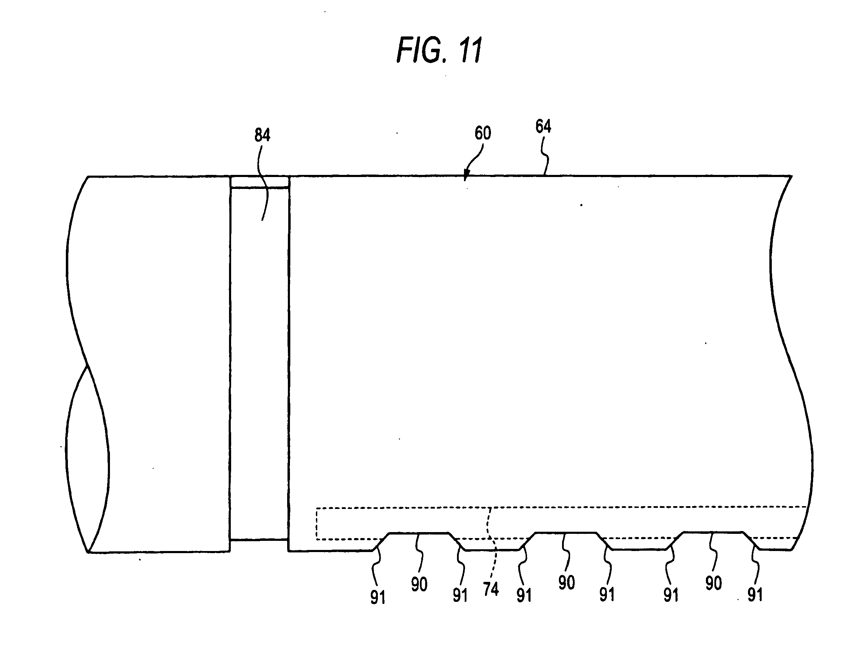

Further, as shown in Fig. 11, the edges 91, 91 on the outer periphery side of each of the openings 90 of the insertion assisting tool 60 according to the embodiment are subjected to the chamfering processing. Thus, as shown in Fig. 9, since the intestinal wall 122 can be prevented from being caught by the openings 90, the operability of inserting and extracting the insertion assisting tool 60 with respect to the intestinal wall 122 can be improved. Further, pain of a patient due to the catch of the intestinal wall 122 by the opening 90 can be relieved. Each of the openings 90may be subjected to the chamfering processing at least at edges 91, 91 which are almost orthogonal to the insertion and extracting direction of the insertion assisting tool 60.

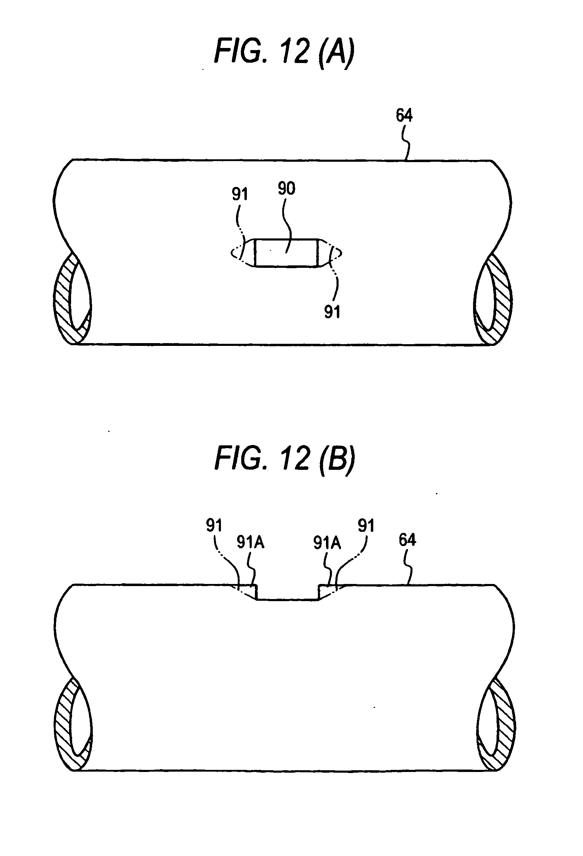

As to the processing method of the opening 90, firstly as shown by a steady line in Fig. 12A, an opening 90 of a rectangular shape is formed at the tube main body 64 by using an ultrasonic cutter. Then, as shown in Fig. 12B, right-angle corner portions 91A, 91A of the edges almost orthogonal to the extracting direction are cut by a cutting means such as a cutter, whereby the opening 90 having the edges 91 subjected to the chamfering processing can be provided.

As explained above, according to the embodiment, since the tube main body 64 of the insertion assisting tool 60 is configured by the multi-lumen tube integrally formed by the insertion path 70 and the tubes 72. 74, the entire flexibility of the insertion assisting tool 60 can be improved as compared with a case where the insertion path 70 and the tubes 72. 74 are separately formed by respective tubes and then pasted from one another. Thus, the operability at the time of inserting the insertion portion 12 of the endoscope 10 and the operability at the time of inserting the insertion assisting tool 60 within a human body can be improved.

Although in the aforesaid embodiment, the tube 72 and the tube 74 are closed at the fixing position of the balloon 66, the closing position is not limited thereto, and these tubes may be closed on the tip end side than the opening 80 and the opening 90. Thus, these tubes may be formed in adjacent to the openings 80, 90, for example. Further, the tubes 72, 74 may be closed only by the tip end portion of the tube main body 64. In this case, the closing process may be performed simultaneously at the time of subjecting the tip end of the tube main body 64 to the taper processing.

Further, although in the aforesaid embodiment, the tube 72 for ventilation is closed at the fixing position of the base end portion 66B of the balloon 66, the invention is not limited thereto, and this tube may be closed only at the fixing position of the tip end portion 66A of the balloon 66.

Furthermore, although in the aforesaid embodiment, the tubes 72, 74 are closed by pressing the tube main body 64 from the outside and by being subjected to the heat processing, the invention is not limited thereto, and these tubes may be closed by filling adhesive etc., for example, within the tubes 72, 74.

Although the aforesaid embodiment shows an example of the single balloon endoscope in which the balloon 66 is attached only to the insertion assisting tool 60, the endoscope may be configured as a double balloon type endoscope in which a freely inflatable balloon is also attached to the insertion potion 12 of the endoscope 10. In this case, the tip end of the insertion portion 12 can be inserted into the deep portion of an intestine by repeatedly performing the insertion operation for inserting the insertion potion 12 of the endoscope 10, the fixing operation for inflating the balloon on the insertion potion 12 side to thereby fix the insertion potion 12, the pushing operation for pushing the insertion assisting tool 60 along the insertion potion 12, the gripping operation for inflating the balloon 66 to thereby grip the intestine and the drawing operation for drawing the insertion assisting tool 60.

## Claims

1. An insertion assisting tool (60) comprising: a multi-lumen tube (64) including a tip end surface, a base end surface and an outer peripheral surface; and a balloon (66) attached to an outer periphery of a tip end portion of the multi-lumen tube, wherein
the multi-lumen tube includes an insertion path (70), which penetrates from the tip end surface to the base end surface and into which an insertion portion of an endoscope is Insertable, and a fluid tube (74) which is provided separately from the insertion path and penetrates from the tip end surface to the base end surface along the insertion path,
the multi-lumen tube has an opening (90) formed at the outer peripheral surface closer to the base end surface than the position where the balloon is disposed, so as to communicate the outer peripheral surface with the fluid tube in the multi-lumen tube,
such that the air or liquid (118) reserved between the multi-lumen tube and
the intestinal wall can be prevented from being compressed when moving the insertion assisting tool in the extracting direction in the inflated state of the balloon (66), and
the fluid tube (74) is closed on the side of the multi-lumen tube which is closer to the tip end than the position of the opening, **characterised in that**
the opening (90) has an elongated shape, and the longitudinal direction of the elongated shape is along the axial direction of the fluid tube (74),
and at least an edge (91) of the opening (90) is chamfered by a chamfering process.

2. The insertion assisting tool according to claim 1, wherein the fluid tube (74) is closed by pressing the tube of the multi-lumen tube from the outside and by subjecting the tube to a heat processing

3. The insertion assisting tool according to claim 1, wherein the fluid tube (74) is closed at a fixing position where a tip end of the balloon (66) is fixed.

4. The insertion assisting tool according to claim 3, wherein the outer peripheral surface is provide with a concave portion (82) at a position where the fluid tube is closed, and the concave portion locates at the fixing position where the tip end of the balloon (66) is fixed.

5. The insertion assisting tool according to claim 1, wherein the position where the fluid tube (74) is closed locates closer to the base end surface than the position where the balloon (66) is disposed.

6. The insertion assisting tool according to claim 1, wherein the multi-lumen tube has a plurality of openings (90), spaced along the fluid tube (74).

## Patentansprüche

1. Einführhilfe (60), umfassend einen Mehrlumen-Schlauch (64), der eine vordere Stirnfläche, eine Basisstirnfläche und eine Außenumfangsfläche enthält, außerdem einen an einem Außendurchmesser eines vorderen Endbereichs des Mehrlumen-Schlauchs befestigten Ballon (66), wobei
der Mehrlumen-Schlauch einen Einführweg (70), welcher von der vorderen Stirnfläche zu der Basisstirnfläche verläuft, und in den ein Einführabschnitt eines Endoskops einführbar ist, und einen Fluidschlauch (74) enthält, der separat von dem Einführweg vorgesehen ist und sich von der vorderen Stirnfläche zu der Basisstirnfläche entlang dem Einführweg erstreckt,
wobei der Mehrlumen-Schlauch eine Öffnung (90) aufweist, die an der Außenumfangsfläche näher an der Basisstirnfläche als an der Stelle, an der sich der Ballon befindet, ausgebildet ist, um die Außenumfangsfläche mit dem Fluidschlauch in dem Mehrlumenschlauch in Verbindung zu bringen,
so dass die Luft oder die Flüssigkeit (118), die zwischen dem Mehrlumen-Schlauch und der Darmwand eingeschlossen ist, an einem Komprimieren gehindert werden kann, wenn die Einführhilfe in Ausziehrichtung bei aufgeblähtem Zustand des Ballons (66) bewegt wird, und der Fluidschlauch (74) auf der Seite des Mehrlumen-Schlauchs, die näher an der vorderen Stirnfläche liegt als an der Stelle der Öffnung, geschlossen ist,
**dadurch gekennzeichnet, dass** die Öffnung (90) eine längliche Form hat und die Längsrichtung der länglichen Form entlang der axialen Richtung des Fluidschlauchs (74) verläuft, und mindestens eine Kante (91) der Öffnung (90) durch einen Abfasungsprozess abgefast ist.

2. Einführhilfe nach Anspruch 1, bei der der Fluidschlauch (74) verschlossen ist durch Pressen des Schlauchs des Mehrlumen-Schlauchs von außen und durch Wärmebehandlung des Schlauchs.

3. Einführhilfe nach Anspruch 1, bei der der Fluidschlauch (74) an der Fixierstelle, an der ein vorderes Ende des Ballons (66) fixiert ist, verschlossen ist.

4. Einführhilfe nach Anspruch 3, bei der die Außenumfangsfläche mit einem konkaven Bereich (82) an einer Stelle ausgestaltet ist, an der der Fluidschlauch geschlossen ist, und der konkave Bereich sich an der Fixierstelle befindet, an der das vordere Ende des Ballons (66) fixiert ist.

5. Einführhilfe nach Anspruch 1, bei der die Stelle, an der der Fluidschlauch (74) geschlossen ist, sich näher an der Basisstirnfläche als an der Stelle befindet, an der sich der Ballon (66) befindet.

6. Einführhilfe nach Anspruch 1, bei der der Mehrlumen-Schlauch eine Mehrzahl von entlang des Fluidschlauchs (74) beabstandeten Öffnungen (90) besitzt.

## Revendications

1. Outil d'aide à l'insertion (60) comportant : un tube à ouverture multiple (64) comprenant une surface d'extrémité terminale, une surface d'extrémité de base et une surface périphérique extérieure ; et un ballonnet (66) fixé sur une périphérie extérieure d'une partie d'extrémité terminale du tube à ouverture multiple, dans lequel le tube à ouverture multiple comprend un passage d'insertion (70), qui pénètre depuis la surface d'extrémité terminale jusqu'à la surface d'extrémité de base et dans lequel une partie d'insertion d'un endoscope peut être insérée, et un tube de fluide (74) qui est prévu séparément du passage d'insertion et pénètre depuis la surface d'extrémité terminale jusqu'à la surface d'extrémité de base le long du passage d'insertion,
le tube à ouverture multiple a une ouverture (90) formé au niveau de la surface périphérique extérieure plus près de la surface d'extrémité de base que la position où le ballonnet est disposé, de façon à faire communiquer la surface périphérique extérieure avec le tube de fluide dans le tube à ouverture multiple,
de telle sorte que l'air ou le liquide (118) réservé entre le tube à ouverture multiple et la paroi intestinale peut être empêché d'être comprimé lors du déplacement de l'outil d'aide à l'insertion dans la direction d'extraction dans l'état gonflé du ballonnet (66), et
le tube de fluide (74) est fermé sur le côté du tube à ouverture multiple qui est plus près de l'extrémité terminale que la position de l'ouverture,
**caractérisé en ce que**
l'ouverture (90) a une forme allongée, et la direction longitudinale de la forme allongée est le long de la direction axiale du tube de fluide (74),
et au moins un bord (91) de l'ouverture (90) est chanfreiné par un processus de chanfreinage.

2. Outil d'aide à l'insertion selon la revendication 1, dans lequel le tube de fluide (74) est fermé en appuyant sur le tube du tube à ouverture multiple depuis l'extérieur et en soumettant le tube à un traitement thermique.

3. Outil d'aide à l'insertion selon la revendication 1, dans lequel le tube de fluide (74) est fermé au niveau d'une position de fixation où une extrémité terminale du ballonnet (66) est fixée.

4. Outil d'aide à l'insertion selon la revendication 3, dans lequel la surface périphérique extérieure est pourvue d'une partie concave (82) dans une position où le tube de fluide est fermé, et la partie concave se trouve dans la position de fixation où l'extrémité terminale du ballonnet (66) est fixée.

5. Outil d'aide à l'insertion selon la revendication 1, dans lequel la position où le tube de fluide (76) est fermé se trouve plus près de la surface d'extrémité de base que la position où le ballonnet (66) est disposé.

6. Outil d'aide à l'insertion selon la revendication 1, dans lequel le tube à ouverture multiple a une pluralité d'ouvertures (90) espacées le long du tube de fluide (76).
